# EUROPEAN PATENT SPECIFICATION

(11) **EP 2 184 108 B1**
(45) Date of publication and mention of the grant of the patent: **04.04.2012**
(21) Application number: 09172215.7
(22) Date of filing: 05.10.2009
(51) Int. Cl.: B01L 3/00, B01L 3/02

(54) **Sample carrier for securing microbiological, virological, genetic, medical veterinary medica, forensic, criminalistic and technical samples**
Probenträger zur Sicherung von mikrobiologischen, virologischen, genetischen, medizinischen, tiermedizinischen, forensischen, kriminalistischen und technischen Proben
Transporteur d'échantillons pour la sécurisation d'échantillons microbiologiques, virologiques, génétiques, médicaux, vétérinaires, médicolégaux, criminalistiques et techniques

(30) Priority: 04.10.2008 DE 202008013218 U
(43) Date of publication of application: 12.05.2010
(73) Proprietor: EPPENDORF AG, 22339 Hamburg (DE)
(72) Inventor: Schmiedl, Dieter, 04626 Schmölln (DE)
(74) Representative: Straus, Alexander

(56) References cited:
- EP-A- 0 928 969
- WO-A-2008/103828
- US-A1- 2005 016 921
- US-A1- 2005 220 677

## Description

### Field of Application

The invention relates to a sample carrier based on a micro litre pipette tip. Said sample carrier is provided for securing evidence in microbiological, virological, genetical, medical, veterinary medical, forensic, criminalistic and technical fields and is designed as a component of an apparatus systick for sampling, identifying, storing and further processing, and providing of samples to be used as evidence, respectively.

### State of the art

In scientific, clinical, veterinary, technical or other research, development or routine laboratories so called micro litre pipette tips of different style and size have been used for many years. Their construction normally depends on the fix volume, range of volume or specific application.

All pipettes have in common that they can be tipped/equipped with one or more exchangeable tips depending on the application purpose. In practice micro litre pipette tips merely serve as pipetting aids. They are in most cases provided for single use and are discharged after use. Depending on the media to be pipetted and the required accuracy pipettes and their respective tips are harmonized.

Collecting systicks with cotton wool swabs are prevailing as actual sample carriers in forensics and criminalistics. These swabs mostly consist of a shaft/stick made of wood, metal or plastics to which a piece of cotton wool is provided on one or both ends. A sample can be taken from a substrate by rubbing off or wiping. The sample may then be further processed.

The reason for it being that DNA is a sensible analytic material, which degrades quickly under moist storage conditions. Drying of the drawn samples is complicated, since contaminations by foreign DNA or a mixing up of samples must be avoided unconditionally. If the cotton wool carrier is not allowed to dry immediately after sample collection the original samples will be spoiled after only a short time. Most of the swabs/cotton wool swabs having a long shaft are normally disposed of in closable sample glasses or cardboard boxes and then sealed. The required space is disproportionally high when considering that only the small amount of cotton actually functions as sample carrier. Some users therefore break off the front cnd of the swabs used. The thus required predetermined breaking point in the swab shaft, however, makes the whole swab mechanically instable.

A further fundamental drawback of conventional sample carriers in form of swabs is that they cannot be directly inscribed and cannot be provided with a barcode, respectively. It is rather accepted to store swabs in labelled test tubes or boxes until further processing. The risk of mixing up samples is very high after removal of the swab from its temporary storage.

German utility model DE 20 2007 001 898 for example describes a container and medical swab for biological materials.

EP 1 234 165 presents a sample container for conservation and dry storage of DNA/RNA containing material. < 2a >

It would be desirable to provide a sample collecting system having an appropriate sample carrier which will not exhibit the identified problems of wetting und drying as well as safe storage and labelling of the samples.

### Problem to be solved

The problem to be solved therefore is to be seen in the development of a sample carrier, particularly for forensic and criminalistic purposes.
EP 0 928 969 relates to a disposable to be applied to e.g. a blood glucose meter. The disposable is a hollow frustrum of a cone and has a membrane attached to the smaller end, which membrane substantially closes the smaller open end.
US 2005/0016921 refers to extrication columns for purifying an analyte from a sample solution for subsequent chemical analysis. The columns may be pipette tip based or may be attached to a pipette tip and harbor in their container an extrication media, held in place and sealed from the environment through membranes.
WO 2008/103828 discloses a pipette tip device for extracting samples to be analyzed. The pipette tip is provided with a solid phase sorbent for extraction purposes. A proximate screen or fit provided in the pipette tip is reported to be composed of a liquid permeable membrane serving as filter unit.

### Solution to the problem

The problem has been solved by the present invention which provides a sample carrier according to claim 1.

In another embodiment of the invention the sample collector is formed as swab tip, as brush tip or as roll.

In still another embodiment of the invention the roll is a cylindrically formed pipette shoulder covered with tissue, blotting paper or a special carrier.

In another embodiment of the invention the sample collector is a combination of a swab-tip, brush tip and roll, respectively.

In still another embodiment of the invention a barcode, a transponder or the like is attached to the micro litre pipette tip.

According to the invention it is proposed to redesign micro litre pipette tips known per se so that they can be applied as sample carrier for the indicated application.

The actual sample collector is therefore directly attached to the opening of the outlet of the micro litre pipette tip. The sample collector is formed as swab tip, as brush tip for turning up hair, textile fibers, feathers, fuzzes or the like (brush tip) or as a turnable cylindrically formed pipette shoulder being covered with tissue, blotting paper or a special carrier and serves for collecting samples by rolling (roller tip).

Combinations of two or all mentioned embodiments are also feasible. The sample collector can be wetted through said opening from the outside and the inside.

According to the invention the sample carriers formed as micro litre pipette tips can be clipped on simple tip holders, conventional micro litre pipettes or medical tips. They can also be preserved in properly dimensioned dry tubes if required.

It is also possible to attach a barcode, a transponder or the like to the outer surface of the sample carrier.

Such further developed micro litre pipette tips can be applied advantageously for taking forensic or microbiological samples. Additionally, they are suitable for transport and preserving for long time periods.

It is proposed to use constructively adapted pipettes for sample collection and sample drawing for an optimal handling of the sample carriers. When being analyzed in the lab the sample carriers can be further processed with the micro litre pipettes present in the laboratory without any problem.

### Embodiments

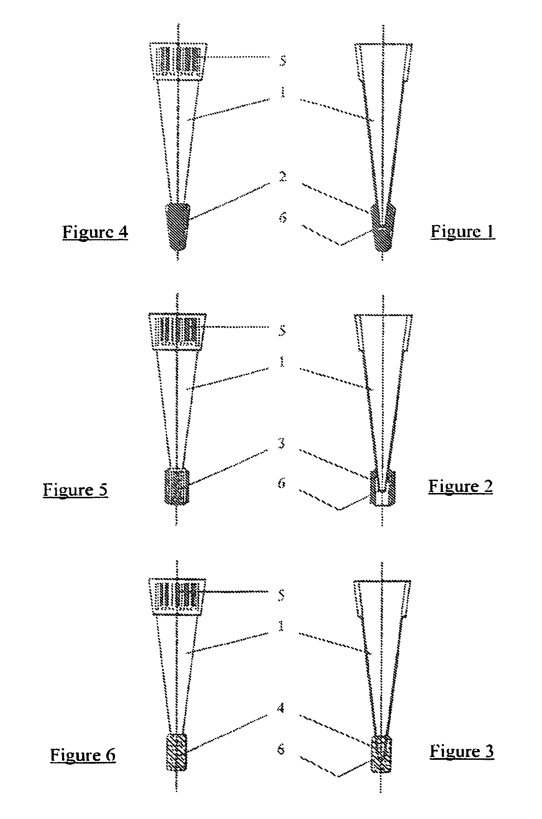

the following embodiments of the invention relate to sample carriers according to the invention, in which the sample collectors are formed as swab tips (Fig. 1), brush tips (Fig. 2), or roller tips (Fig. 3). In Fig. 4, Fig. 5 and Fig. 6 these tips are each presented with applied barcode. Alternatively, also a transponder or another advantageous labelling can be used.

The reference signs used in the drawings have the following meaning:

| | |
|---|---|
| 1 | micro litre pipette tip |
| 2 | cotton wool wad (swab tip) |
| 3 | micro brush (brush tip) |
| 4 | roll (roller tip) |
| 5 | barcode, transponder |
| 6 | opening. |

As can be seen in all the figures shown a micro litre pipette tip 1 of conventional style having technical means for being attached to a tip holder is a conventional micro litre pipette or a medical formed tip and having an opening 6on the pointed opposite outlet.

Alternatively, a swab tip 2, a brush tip 3, and a roll 4 covered a with a tissue, a blotting- or special paper, respectively, is appropriately disposed, but always disposed in such a way in the region of said opening 6 that said mentioned technical means can be wetted from the inside through said opening 6.

Combinations of swab tip 2, brush tip 3, and roll 4 are possible and in certain cases reasonable.

The micro litre pipette tip I can be provided with a bar code 5. transponder 5 or the like in any embodiment, namely as swab tip, brush tip and roller tip. Sample carriers formed as swab tips, i.e. micro litre pipette tip 1 with applied swab tip 2, are full-fledged smear swabs and suited at best for tasks in microbiology, genetics or forensics.

Said sterile swab tip 2 consists normally of cotton, viscose, alginate, vinyl alcohol or any other material suitable for the operation purpose. Said swab serves for collecting all kinds of samples, for example germs, saliva, sweat, smallest blood amounts, semen, secretions or cosmetics.

Wetting of said cotton wool is always necessary when DNA samples are rubbed off from dry or absorptive substrates.

Said swab tips can be wetted conservatively by means of a dropping bottle analogously to the known cotton-shaft swabs. When inserting correspondingly constructed collecting pipettes (forensipettes/forensic pipettes) they can be wetted conveniently from the inside, such that further wetting is possible without any problem even during rubbing off a sample. Depending on the desired use swab tips are recommended in different sizes and, as already common in the lab, in clearly distinguishable colour design, for example white, yellow or blue, having swab tips 2 with varying collecting capacity. Thin swab tips can, for example, be applied for collecting dirt samples from finger nails.

The brush tip 3 is designed for collecting hair, textile fibers, feathers or fuzzes by rotational movements.

The roll 4 is a cylindrically formed pipette shoulder which is covered with tissue, blotting paper or a special carrier, for example FTA-paper from the company Whatman Inc., and is intended to be used in collecting samples by rolling. Wetting takes place as described above.

The barcodes 5 or transponders 5 are advantageously mounted from outside on the upper part of the micro litre pipette tip 1 and thus do not contact the sample. The samples can therefore be clearly assigned.

In their function as sample carriers said swab tips, brush tips or roller tips can be stored contact-free in dry tubes or other suitable containers. Adhering samples can thus be preserved for a long time period or are available for microbiological purposes when stored in the upper part of the micro litre pipette tip 1 and thus do not contact the sample. The samples can therefore be clearly assigned.

In their function as sample carriers said swab tips, brush tips or roller tips can be stored contact-free in dry tubes or other suitable containers. Adhering samples can thus be preserved for a long time period or are available for microbiological purposes when stored in a culture medium. Even demanding microbiological germs can be safely transported if introduced into respective transport media. The sample carriers according to the invention are suitable either for dry as well as wet microbiological smears. Within the technical area of securing DNA-samples said sample carriers can be excellently applied for forensic smears of the oral mucosa for relationship and paternity studies.

By said already mentioned storage of used sample carriers in tightly sealing containers having drying agents biological samples, for example DNA samples, can be preserved even without previous analysis directly over long time periods in the line with DNA-banking and can be extracted for examination at a future date. Extraction of the sample carriers should advantageously be done contamination-free by means of conventional laboratory pipettes.

Said attached barcode 5 or transponder 5 provides a high level of security from mixing up samples. Also other advantageous labellings can be applied.

## Claims

1. Sample carrier in form of a micro litre pipette tip (1) for securing microbiological, virological, genetic, medical, veterinary medical, forensic, criminalistic and technical samples, having technical means for being attachable to a tip holder, a conventional micro litre pipette or a medical syringe and having an opposite pointed outlet with an opening (6), **characterized in that** a sample collector is formed as swab tip (2), as brush tip (3) or as a roll (4) and is attached in such a way directly in the region of said opening (6), that wetting of the sample collector is feasible from the inside of the micro litre pipette tip (1) through said opening (6).

2. The sample carrier according to claim 1, wherein the roll (**4**) is a cylindrically formed pipette shoulder covered with tissue, blotting paper or a FTA Paper.

3. The sample carrier according to claim 1, **characterized in that** a barcode (5), a transponder (5) or the like is attached to said micro litre pipette tip (1).

## Patentansprüche

1. Probenträger in Form einer Mikroliterpipettenspitze (1) für die Sicherung von mikrobiologischen, virologischen, genetischen, medizinischen, veterinärmedizinischen, forensischen, kriminaltechnischen und technischen Proben, mit technischen Mitteln zum Anbringen an einen Spitzenhalter, eine herkömmliche Mikroliterpipette oder eine medizinische Spritze und mit einem gegenüberliegenden, spitz zulaufenden Auslauf mit einer Öffnung (6), **dadurch gekennzeichnet, dass** unmittelbar im Bereich der Öffnung (6) eine Probenaufnahme als Wattetupfer (2), als Mikrobürste (3) oder als eine Rolle (4) derart ausgebildet ist, dass ein Befeuchten der Probenaufnahme aus dem Inneren der Mikroliterpipettenspitze (1) durch die Öffnung (6) hindurch realisierbar ist.

2. Probenträger nach Anspruch 1, wobei die Rolle (4) ein mit Gewebe, Fließpapier oder einem FTA-Papier bezogener, zylinderartig geformter Pipettenansatz ist.

3. Probenträger nach Anspruch 1, **dadurch gekennzeichnet, dass** an der Mikroliterpipettenspitze (1) ein Barcode (5), ein Transponder (5) oder dergleichen angebracht ist.

## Revendications

1. Dispositif de transport d'échantillon sous la forme d'un embout de pipette microlitre (1) pour la sécurisation d'échantillons microbiologiques, virologiques, génétiques, médicaux, de médecine vétérinaire, de médecine légale, criminalistiques et techniques, ayant un moyen technique pour pouvoir être fixé sur un support d'embout, une pipette microlitre classique ou une seringue médicale et ayant une sortie pointue opposée avec une ouverture (6), **caractérisé en ce qu'**un collecteur d'échantillon est formé sous la forme d'un embout d'écouvillon (2), d'une pointe de pinceau (3) ou d'un rouleau (4) et est fixé de telle manière directement dans la région de ladite ouverture (6), que le mouillage du collecteur d'échantillon est réalisable depuis l'intérieur de l'embout de pipette microlitre (1) à travers ladite ouverture (6).

2. Dispositif de transport d'échantillon selon la revendication 1, dans lequel le rouleau (**4**) est un épaulement de pipette en forme de cylindre couvert avec un tissu, un papier buvard ou un papier FTA.

3. Dispositif de transport d'échantillon selon la revendication 1, **caractérisé en ce qu'**un code à barres (5), un transpondeur (5) ou autre est fixé sur ledit embout de pipette microlitre (1).
